Europäisches Patentamt

European Patent Office

Office européen des brevets

(11) Publication number: **0 384 657**
**A1**

## (12) EUROPEAN PATENT APPLICATION

(21) Application number: **90301673.1**

(22) Date of filing: **15.02.90**

(51) Int. Cl.5: **B29C 45/00, B29C 45/26, A61M 5/31**

(30) Priority: **16.02.89 GB 8903581**

(43) Date of publication of application:
**29.08.90 Bulletin 90/35**

(84) Designated Contracting States:
**AT BE CH DE DK ES FR GB GR IT LI LU NL SE**

(71) Applicant: **CMB PACKAGING (UK) LIMITED**
**Woodside Perry Wood Walk**
**Worcester WR5 1EQ(GB)**

(72) Inventor: **De'Ath, Roderick Michael**
**18 Haywards Close**
**Wantage, Oxon OX12 7AT(GB)**
Inventor: **Griffiths, Donald Ernest**
**72 Uppermanor Road, Preston**
**Paignton, S. Devon, TQ3 2TJ(GB)**
Inventor: **Hodkinson, Andrew**
**35 Church Road**
**Banks, Southport PR9 j8ET(GB)**

(74) Representative: **Lerwill, John et al**
**A.A. Thornton & Co. Northumberland House**
**303-306 High Holborn**
**London, WC1V 7LE(GB)**

(54) Injection moulding.

(57) A hollow article, especially a syringe body, having a tubular portion (1) and a flange (4) is injection moulded in a mould having a single injection orifice located at the extremity of the mould cavity part which defines the flange. The mould cavity has a pair of channels which define ribs on the flange of the syringe body and which have substantial equal length and area for conducting molten material simultaneously to opposite sides of the cavity part which forms the tubular portion.

## Injection Moulding

This invention relates to the field of injection moulding, and it refers in particular to the manufacture by injection moulding of articles made of thermoplastics and comprising a hollow portion. For convenience the invention will be described herein with specific reference to the production of syringe bodies, but the principles underlying the invention are applicable equally to other articles of generally similar form.

A known syringe body of integral construction and made as an injection moulding of a thermoplastics material, comprises a hollow cylindrical barrel with a forward end wall having a nozzle projecting axially therefrom, the rear end of the barrel being open to receive the syringe piston, and an external flange at the rear end of the barrel defining a pair of opposed wings extending laterally from the barrel to enable the body to be held by the first and second fingers of one hand while forcing the piston forwards with the thumb of the same hand to discharge the syringe contents through the nozzle. The known syringe body is produced in a mould having a mould cavity which the molten thermoplastics material enters via a pair of injection orifices or gates located diametrically opposite one another at a medial position along the barrel length. During the moulding process it is important that the thermoplastics should enter essentially simultaneously through the two injection orifices. Because the material is forced into the mould under high pressure, if one orifice opens before the other the mould parts are subjected to large out of balance forces with the result that they can be moved relative to one another causing the finished moulding to have a non-uniform thickness around the barrel or to be defective in some other way due to uneven flow emanating from the two orifices so that it has to be discarded. The exact timing of the opening of the injection orifices is difficult to control, with the consequence that relatively large wastages of moulded syringes bodies can be suffered.

In EP-A-0315586, for example, there is shown an injection mould for producing syringe bodies in which moulding material is injected into the mould cavity through at least two orifices uniformly distributed around the mould cavity and located axially at the level of the forward end wall connecting the barrel to the nozzle. US-A-4184836 refers to a multicavity mould for production of syringe bodies with each cavity having a single radial injection orifice also at the axial position of the end wall between the barrel and nozzle, the cavity portion defining the end wall being dimensioned and shaped non-symmetrically about the axis to ease entry of molten material and distribution thereof in the mould cavity. Neither of these proposals provides an effective answer to the problems mentioned.

With the basic objective of providing a solution to the problem explained above, in accordance with one aspect the invention resides in a method of manufacturing by injection moulding an article comprising a hollow portion with an external flange connected thereto, the method comprising filling a mould cavity having the shape of said article with thermoplastic material, the material being injected into the cavity through a single orifice opening into a first cavity part defining the shape of said flange, said first cavity part including a pair of channels of substantially equal length and flow cross-section leading from the injection orifice and connecting to a second cavity part defining the shape of the hollow portion, and said channels opening into the second cavity part defining the shape of the hollow portion at respective positions on opposite sides thereof, whereby molten material is conducted into said second cavity part substantially simultaneously via the two channels.

In accordance with another aspect the invention provides an injection moulded article comprising a tubular portion having an external flange, the flange having a pair of symmetrical ribs extending from a common point at the outer edge of the flange to respective points of intersection with the tubular part at generally diametrically opposed locations, said ribs being constituted by runners which supply material to form the tubular portion during manufacture of the article.

According to a third aspect the invention provides an injection mould comprising a plurality of mould parts which when closed together define a mould cavity including one part for forming a hollow article portion and an adjoining part for forming an external flange on the hollow portion, a single injection gate located at the outer extremity of said adjoining cavity part, and said adjoining cavity part including a pair of channels each extending from said gate to said one cavity part, said channels being of substantially equal length and area and being arranged to conduct moulding material into the said one cavity part substantially simultaneously at opposite sides thereof.

Reverting to the specific example of a syringe body, the flange at the rear end of the body provides a convenient vehicle for delivering molten plastics for forming the barrel during the injection moulding process. The injection orifice is conveniently located at the radially outer edge of one of the two wings formed by the flange, and a pair of ribs defining the peripheral edge of this wing extend from the point of injection to diametrically opposed locations at the rear end of the barrel. Thus, the ribs serve the function of runners for delivering molten thermoplastic material into that part of the mould cavity defining the syringe barrel during the moulding process, and in this way it is ensured that the molten material enters and fills the mould cavity without risk of subjecting the mould parts to unacceptably high out of balance forces.

To assist a clear understanding of the invention a more detailed description is given below with reference to the accompanying drawings in which:-

Figure 1 is a side elevation of a syringe body embodying the invention;

Figure 2 is an enlarged cross section taken along the line II-II in Figure 1; and

Figure 3 is a partial cross section taken along the line III-III in Figure 2;

Figure 4 is a top view of the syringe body shown in Figure 1;

Figure 5 is a front end view of the syringe body shown on an enlarged scale;

Figure 6 is a rear end view of the syringe body shown on an enlarged scale;

Figures 7 and 8 are views corresponding to Figs. 5 and 6 and showing a second embodiment of a syringe body;

Figures 9 and 10 are views corresponding to Figs. 5 and 6 and showing a third embodiment of a syringe body; and

Figures 11 and 12 are views corresponding to Figs. 5 and 6 and showing a fourth embodiment of a syringe body.

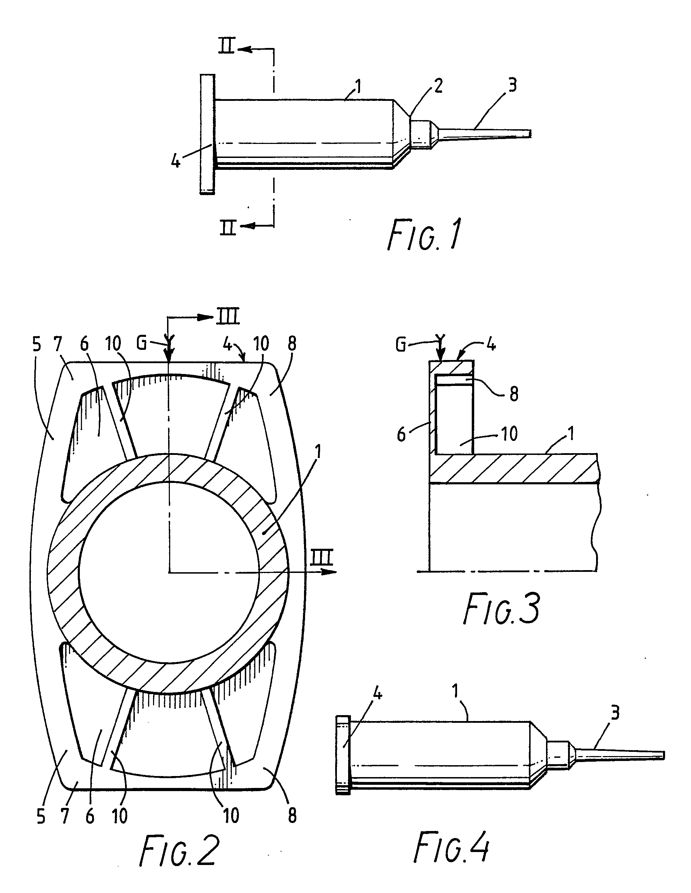

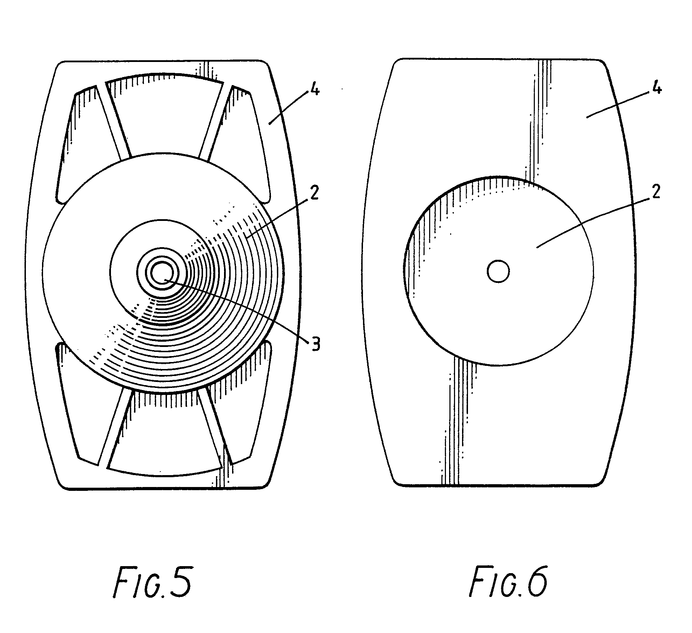

Illustrated in Figures 1 to 6 of the drawings is a syringe body of one-piece injection moulded construction, e.g. of polyethylene. The body includes a cylindrical barrel 1 with a forward end wall 2 from which extends axially a nozzle. The rear end of the barrel is open for receiving the syringe piston which forms no part of the invention and does not require description as it may be of conventional form. Integral with the rear end of the barrel is an external flange 4 shaped to define two diametrically opposed radially projecting wings 5 of symmetrical configuration. As apparent from the drawings, each wing has a very thin flat portion 6 level with the end of the barrel and a peripheral rim of substantially greater thickness defining a pair of ribs 7, 8 extending from the radial mid-plane of the wing to the barrel which the ribs adjoin at diametrically opposite locations. Each rib is integral with the barrel over an angle of around 30° - 40° around the barrel axis. In order to reinforce further the flat portion 6 of the wing, radial webs 10 may interconnect the barrel and the ribs as shown. The webs may have the same depth as the ribs although they could have a smaller depth if preferred.

The syringe body is made in an injection mould defining a cavity of the same shape as the body when the mould parts are closed together. Thus, one part of the cavity will be shaped to form the flange and another adjoining part of the cavity will be shaped to form the barrel, a still further part being shaped to form the end wall and nozzle. The mould is provided with a single gate for injecting molten plastics into the mould cavity, this gate being located at the outer edge of one wing 5 and on the axial mid-plane, ie. at the position indicated by the arrow G in the drawings. The said one part of the mould cavity includes channels which are shaped to form the respective ribs 7, 8, but these channels also serve as runners during the moulding process for conducting the molten plastics into the part of the cavity shaped to form the barrel 1.

The rib channels define two equal flow paths of increased flow area compared with the remaining parts of the one cavity part whereby, when the molten plastic is injected through the gate under high pressure it flows preferentially through these channels into the other cavity parts. Thus, the plastics enters the barrel forming part of the cavity essentially simultaneously at two substantially diametrically opposite locations whereby out-of-balance radially forces on the mould core are precluded.

It will be understood that modifications are possible without departing from the fundamental concept. For example, the rim of the flange could project to the rear of the flat portion or if desired the flat portion could be at any intermediate depth with respect to the rim.

The rim of the flange formed by the ribs 7, 8 acts to reinforce the flange allowing the flat portion 6 to be made much thinner than is necessary when no ribs are present.

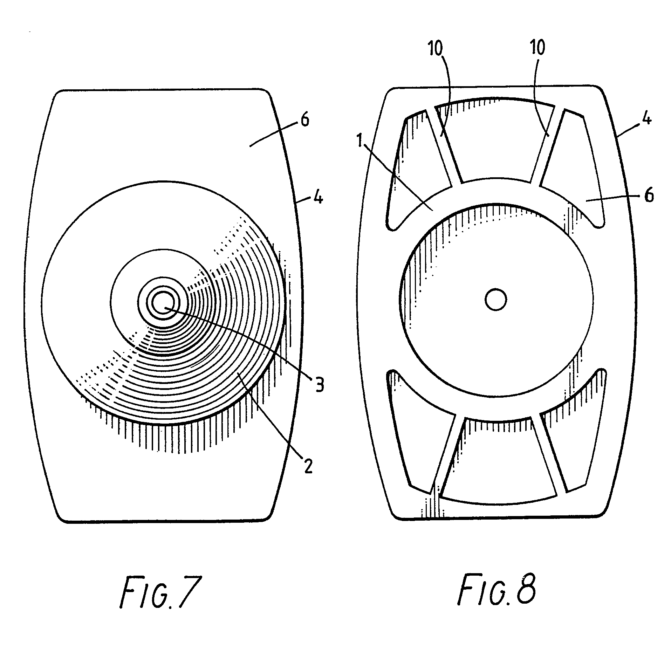

The syringe body of Figures 7 and 8 differs from that of Figures 1 to 6 in that the flange 4 is reversed so that the rim of the flange is directed rearwardly, the rear face of the rim being coplanar with the end of the barrel.

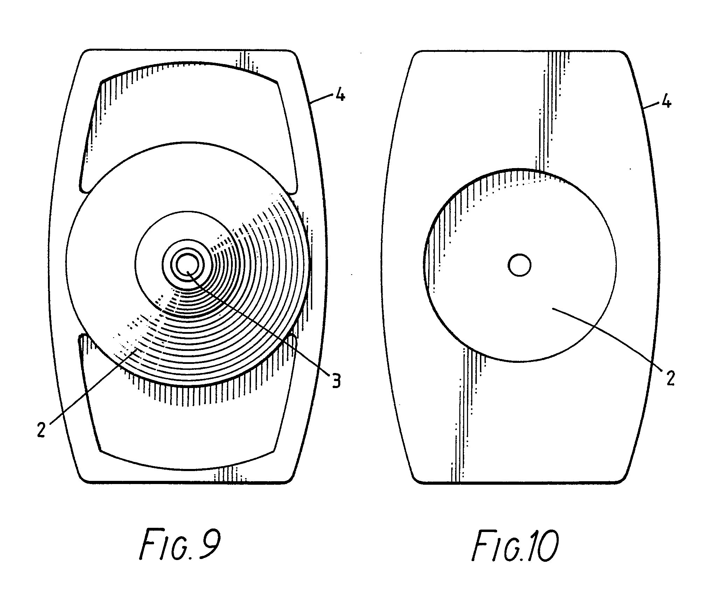

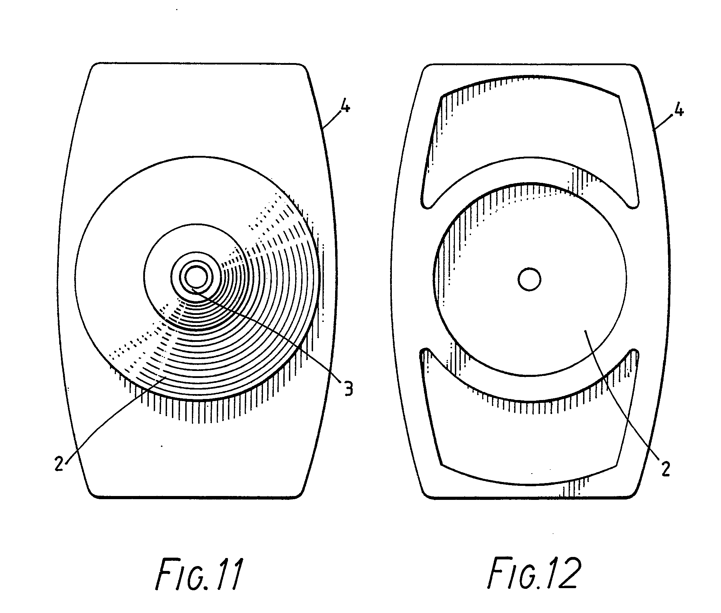

The embodiment of Figures 9 and 10 is identical to that of Figures 1 to 6 except that the stiffening webs 10 are omitted. Similarly, the embodiment of Figures 11 and 12 is identical to that of Figures 7 and 8 except that the stiffening webs 10 are omitted. It will be appreciated that for each of the syringe bodies depicted in Figures 7 to 12, the side elevation and top plan would be as shown in Figures 1 to 4 for the first embodiment.

## Claims

1. A method of manufacturing by injection moulding an article comprising a hollow portion with an external flange connected thereto, the method comprising filling a mould cavity having the shape of said article with thermoplastic material, characterised in that the material is injected into the cavity through a single orifice opening into a first cavity part defining the shape of said flange, said first cavity part including a pair of channels of substantially equal length and flow cross-section leading from the injection orifice and connecting to a second cavity part defining the shape of the hollow portion, and said channels opening into the second cavity part defining the shape of the hollow portion at respective positions on opposite sides thereof, whereby molten material is conducted into said second cavity part substantially simultaneously via the two channels.

2. A method according to claim 1, wherein the channels are arranged to form on the flange a pair of ribs of increased thickness extending in opposite directions along the edge of the flange from the injection orifice.

3. An injection moulded article comprising a tubular portion (1) having an external flange (4), characterised in that the flange has a pair of symmetrical ribs (7, 8) extending from a common point at the outer edge of the flange to respective points of intersection with the tubular part at generally diametrically opposed locations, said ribs being constituted by runners which supply material to form the tubular portion during manufacture of the article.

4. An article as claimed in claim 3, wherein the ribs (7, 8) extend in opposite directions around the peripheral edge of the flange from a central position corresponding to the location of an injection orifice during moulding of the article.

5. An article according to claim 4, wherein the flange includes reinforcing webs (10) interconnecting the tubular portion with the ribs at positions spaced from said central position.

6. An article as claimed in claim 3, 4 or 5, wherein the tubular portion is the barrel (1) of a syringe body, and the flange is a wing (5) at the rear end of the barrel.

7. An injection mould comprising a plurality of mould parts which when closed together define a mould cavity including one part for forming a hollow article portion and an adjoining part for forming an external flange on the hollow portion, characterised in that a single injection gate is located at the outer extremity of said adjoining cavity part, and said adjoining cavity part includes a pair of channels each extending from said gate to said one cavity part, said channels being of substantially equal length and area and being arranged to conduct moulding material into the said one cavity part substantially simultaneously at opposite sides thereof.

8. An injection mould according to claim 7 wherein said channels extend around the periphery of said adjoining cavity part.

EP 0 384 657 A1

FIG.1

FIG.2

FIG.3

FIG.4

FIG.5

FIG.6

FIG.7

FIG.8

FIG.9

FIG.10

*Fig.11*

*Fig.12*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,A D | EP-A-0 315 586 (SCHÖTTLI) <br> * The whole document * <br> --- | 1,3,6,7 | B 29 C 45/00 <br> B 29 C 45/26 <br> A 61 M 5/31 |
| A,D | US-A-4 184 836 (REES) <br> * The whole document * <br> --- | 1,3,6,7 | |
| A | FR-A-2 310 206 (COSTER TECHNOLOGIE SPECIALI) <br> * Page 4, lines 35-39; figures 7,8 * <br> --- | 1,3,7 | |
| A | US-A-2 799 435 (ABPLANALP) <br> * Column 3, lines 16-48; figures 1,3 * <br> --- | 1,3,7 | |
| A | US-A-4 516 969 (KINTNER) <br> * Column 3, lines 11-36; figures 4-8 * <br> ----- | 1,3,6,7 | |

**TECHNICAL FIELDS SEARCHED (Int. Cl.5)**

B 29 C
A 61 M

The present search report has been drawn up for all claims

| Place of search | Date of completion of the search | Examiner |
|---|---|---|
| THE HAGUE | 17-05-1990 | BOLLEN J.A.G. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)